# EUROPÄISCHE PATENTSCHRIFT

(11) **EP 3 070 466 B1**
(45) Veröffentlichungstag und Bekanntmachung des Hinweises auf die Patenterteilung: **13.11.2019**
(21) Anmeldenummer: 16160162.0
(22) Anmeldetag: 14.03.2016
(51) Int. Cl.: G01N 29/032, A61M 1/36, A61M 5/36

(54) **GASBLASEN- UND/ODER FESTKÖRPERDETEKTOR AUF ULTRASCHALLBASIS, DIALYSEGERÄT UND VERFAHREN FÜR EINEN DERARTIGEN DETEKTOR**
GAS BUBBLE AND/OR SOLID OBJECT DETECTOR BASED ON ULTRASOUND, DIALYSIS DEVICE AND METHOD FOR SUCH A DETECTOR
DETECTEUR A ULTRASONS DE CORPS SOLIDES ET/OU DE BULLES DE GAZ, APPAREIL DE DIALYSE ET PROCEDE POUR UN TEL DETECTEUR

(30) Priorität: 17.03.2015 DE 102015103938
(43) Veröffentlichungstag der Anmeldung: 21.09.2016
(73) Patentinhaber: B. Braun Avitum AG, 34212 Melsungen (DE)
(72) Erfinder: Hollstein, Rolf, 36211 Alheim-Heinebach (DE)
(74) Vertreter: Winter, Brandl, Fürniss, Hübner, Röss, Kaiser, Polte - Partnerschaft mbB

(56) Entgegenhaltungen:
- US-A- 5 191 795
- US-A1- 2012 285 870
- US-B1- 6 324 901

## Beschreibung

Die Erfindung geht aus von einem Gasblasen- und/oder Festkörperdetektor auf Ultraschallbasis zum Überwachen eines Mediums. Des Weiteren betrifft die Erfindung e in Dialysegerät mit einem derartigen Detektor und ein Verfahren mit einem derartigen Detektor.

In Dialysegeräten werden üblicherweise Detektoren auf Ultraschallbasis (im Folgenden als Detektor bezeichnet) eingesetzt, um Patienten vor gefährlichen Luftembolien zu schützen. Hierfür kann ein zu überwachender Teil eines extrakorporalen Blutkreislaufes in Form eines Blutschlauchs durch eine Ultraschallstrecke der Detektoren geführt werden. Ein Detektor hat hierzu ein Piezoelement, das durch elektrische Anregung auf seine Resonanzfrequenz einen Ultraschall(US)-Impuls bzw. US-Wellen durch den Blutschlauch sendet. Diesen US-Impuls kann der weitere Detektor in Form eines Piezoelements in ein elektrisches Signal wandeln. Dieses Signal kann durch einen Komparator mit einer Referenzspannung verglichen und bewertet werden. Ausgangsseitig des Komparators steht dann ein Signal zur Verfügung, welches Auskunft darüber gibt, ob sich in der Ultraschallstrecke "Luft" oder "keine Luft" (also nur Flüssigkeit bzw. Blut) befindet.

Gemäß der Veröffentlichung "Bildgebende Verfahren in der Medizin. Von der Technik zur medizinischen Anwendung." von Prof. Dr. Olaf Dössel, Univ. Karlsruhe, ISBN 3-540-66014-3 können US-Impulse das durch den Blutschlauch geführte Blut des Patienten schädigen. Die Schädigung kann hierbei insbesondere erfolgen durch Wärmeeinwirkung und durch Kavitation. Wärme entsteht hierbei lokal proportional zur vom Blut absorbierten Schallintensität. Bei der Kavitation handelt es sich um einen Effekt, bei dem in einer Unterdruckphase eines US-Impulses im Gewebe Gasbläschen entstehen, die dann in einer Druckphase kollabieren.

In der Druckschrift DE 197 381 46 B4 ist ein Ultraschallschwinger offenbart, der auf oder in einer Nähe einer Resonanzfrequenz betrieben wird, um ein starkes Ausgangssignal am Ultraschallschwinger zu erzeugen. Im Einsatz dieses Ultraschallschwingers können die vorstehend beschriebenen Schädigungen beim Blut auftreten.

Die US 2012/285870 A1 offenbart eine Zuführeinrichtung für flüssige Medikamente die den Ultraschall- (US) Energieeintrag begrenzt

Die US 5 191 795 A offenbart eine ultraschallbasierte Flüssigkeits-Erkennungseinrichtung für eine intravenöse Medikamentenzuführeinrichtung an Patienten.

Die US 6 324 901 B1 offenbart ein Verfahren sowie eine Vorrichtung mit dem/der Fremdkörper bei Vorhandensein von stückigen Inhaltsstoffen erkannt und unterschieden werden können. Es handelt sich um eine Anwendung im Lebensmittelbereich.

Demgegenüber liegt der Erfindung die Aufgabe zugrunde, einen Gasblasen- und/oder Festkörperdetektor auf Ultraschallbasis zu schaffen, der mit geringem vorrichtungstechnischem Aufwand sicher einsetzbar ist. Des Weiteren liegt der Erfindung die Aufgabe zugrunde, ein Dialysegerät mit einem Gasblasen- und/oder Festkörperdetektor auf Ultraschallbasis zu schaffen, das einfach ausgestaltet ist und sicher einsetzbar ist. Außerdem ist es Aufgabe der Erfindung ein Verfahren für einen Detektor zu schaffen, mit dem ein zu überwachendes Medium auf vorrichtungstechnisch einfache Weise sicher überwacht werden kann.

Die Aufgabe hinsichtlich des Gasblasen- und/oder Festkörperdetektors auf Ultraschallbasis wird gelöst gemäß den Merkmalen des Anspruchs 1, hinsichtlich des Dialysegeräts gemäß den Merkmalen des Anspruchs 9 und hinsichtlich des Verfahrens gemäß den Merkmalen des Anspruchs 12.

Erfindungsgemäß ist ein Gasblasen- und/oder Festkörperdetektor auf Ultraschallbasis (im Folgenden als Detektor bezeichnet), insbesondere ein Ultraschall-Luftblasen-Detektionssystem oder -einrichtung, zur Detektion von Luft in einem Medium, insbesondere in Blut, vorgesehen. Der Detektor hat einen Ultraschall(US)-Sender zum Senden eines Ultraschalls über bzw. durch das Medium hindurch zu einem Ultraschall(US)-Empfänger des Detektors. Vorteilhafterweise ist/wird ein Energieeintrag in das Medium aufgrund des Ultraschalls überwacht und/oder begrenzt, wofür ein dafür angepasstes Überwachungs- und/oder Begrenzungsmittel bzw. - einrichtung vorgesehen ist.

Diese Lösung hat den Vorteil, dass ein zu hoher Energieeintrag in das Medium erkannt und verhindert werden kann. Wird der Detektor beispielsweise für eine Dialyse eingesetzt, so kann durch den erfindungsgemäßen Detektor eine Schädigung des Bluts durch einen zu hohen Energieeintrag und beispielsweise eine sich hierdurch ergebende Hämolyse vermieden werden. Somit kann das zu überwachende und/oder zu prüfende Medium vor zu hohen Energien bzw. vor einem zu hohen Energieeintrag geschützt werden. Der Energieeintrag auf das zu überwachende Medium wird somit auf ein verträgliches Maß begrenzt. Ein weiterer Vorteil der Überwachung des Energieeintrags liegt darin, dass in Kenntnis der eingetragenen Energie und mithilfe der vom US-Empfänger empfangenen Ultraschalls (Amplitude und zeitlicher Signalverlauf) auf eine Beschaffenheit eines Gegenstands oder eines fremden Mediums in dem zu überwachenden Medium geschlossen werden kann. Handelt es sich beispielsweise um ein bekanntes Medium (Luft) in dem zu überwachenden Medium, so kann mithilfe des empfangenen Ultraschalls sogar auf die Geometrie (Abmessung, Volumen) geschlossen werden.

Die Überwachung des Energieeintrags erfolgt vorrichtungstechnisch einfach durch eine Überwachung einer Ultraschallgenerierung. Die Ultraschallgenerierung wiederum erfolgt durch ein Ansteuersignal. Hierbei kann der US-Sender über eine Steuereinheit (Mikroprozessor, Mikrokontroller, µC) durch das Ansteuersignal gesteuert sein. Zum Überwachen des Energieeintrags kann dann einfach das Ansteuersignal der Steuereinheit abgegriffen werden. Durch die Überwachung der Ultraschallgenerierung kann somit auf vorrichtungstechnisch einfache Weise der Energieeintrag überprüft werden, womit beispielsweise keine zusätzlichen Sensoren notwendig sind.

Das abgegriffene Steuersignal kann vorzugsweise von einer zweiten Steuereinheit (Mikroprozessor, Mikrokontroller, µC) überwacht werden, welche in diesem Fall das Überwachungs- und/oder Begrenzungsmittel darstellt. Hierdurch wird vorteilhafterweise die Überwachung nicht der ersten Steuereinheit überlassen, sondern von einer zweiten zusätzlichen Steuereinheit durchgeführt, wodurch die Sicherheit des Systems erhöht ist, insbesondere wenn die erste Steuereinheit fehlerhaft ist. Die zweite Steuereinheit kann das Steuersignal beispielsweise mit einem Soll-Steuersignal vergleichen und somit einen Mustervergleich durchführen. Es ist denkbar, dass die zweite Steuereinheit bei Bedarf den Energieeintrag durch geeignete Maßnahmen begrenzt.

Mit Vorteil sind der US-Sender (Transducer) und der US-Empfänger (Transducer) durch jeweils zumindest ein Piezoelement ausgebildet. Der US-Sender kann dann zum Aussenden des Ultraschalls mit einer Spannung einer Spannungseinrichtung oder einer Energie eines Energiespeichers beaufschlagt sein. Bei der Spannungseinrichtung handelt es sich beispielsweise um eine elektrisch parallel zum US-Sender angeordnete Induktivität. Alternativ oder zusätzlich ist denkbar, dass es sich bei der Spannungseinrichtung um eine Spannungsverdopplerschaltung (Kaskadenschaltung) handelt. Die Spannungseinrichtung kann auch Schaltungen aufweisen, die dem Prinzip von Schaltreglern folgen.

Alternativ wird ein Burst-Intervall des Ansteuersignals überwacht. Dieses stellt eine Zeitspanne zwischen Startzeitpunkten zweier aufeinander folgender Ladevorgänge der Spannungseinrichtung dar. Bei einer Verringerung des Burst-Intervalls kann auf eine Erhöhung des Energieeintrags in dem Medium geschlossen werden. Das Burst-Intervall wird beispielsweise zur Überwachung mit einem Soll-Burst-Intervall verglichen.

Alternativ oder zusätzlich zum Burst-Intervall kann eine vom Ansteuersignal vorgegebene Ladezeit der Spannungseinrichtung oder die Energie im Energiespeicher überwacht sein. Erhöht sich die Ladezeit der Spannungseinrichtung, so kann von einem höheren Energieeintrag ausgegangen werden. Eine Veränderung kann insbesondere durch Heranziehung einer Soll-Ladezeit festgestellt werden.

Alternativ oder zusätzlich ist denkbar, eine Anzahl vom US-Sender ausgegebene Stimuli (Rechtecksignale, Impulse), die insbesondere vom Ansteuersignal vorgegeben sind, pro Burst-Intervall zu überwachen. Diese können dann mit einer Soll-Anzahl verglichen werden. Erhöht sich die Anzahl der Stimuli, so muss von einem höheren Energieeintrag ausgegangen werden.

Wird eine Veränderung des Burst-Intervalls und/oder der Ladezeit und/oder der Anzahl der Stimuli und/oder der US-Anregungsfrequenz und/oder der Periodendauer festgestellt, so kann ein Fehlersignal, insbesondere von der zweiten Steuereinheit, ausgegeben werden. Die angeführten Veränderungen der jeweiligen Parameter können beispielsweise durch ein temporäres oder dauerhaftes Fehlerverhalten der ersten Steuereinheit (Software- und/oder Hardwarefehler) entstehen.

In weiterer Ausgestaltung der Erfindung wird bzw. werden bei einem fehlerhaften Energieeintrag eine Maßnahme oder mehrere Maßnahmen eingeleitet, die den Energieeintrag begrenzt bzw. begrenzen oder beendet bzw. beenden. Diese Maßnahmen können dabei vorzugsweise von der zweiten Steuereinheit eingeleitet werden, wie z. B. Stoppen der Energiezufuhr zum US-Sender, Stopp von Pumpen und/oder Schließen von Ventilen.

Beispielsweise handelt es sich bei den Maßnahmen insgesamt auch um HardwareMaßnahmen, indem der fehlerhafte Energieeintrag beispielsweise durch ein Bauteil oder mehrere Bauteile (Elektronik-Bauteile, Hardwarekomponenten) begrenzt ist. Des Weiteren ist denkbar, als Hardware-Maßnahme die Ausgestaltung (Dimensionierung bzw. Auswahl) der Induktivität mit einem entsprechenden Sättigungsstrom vorzusehen. Alternativ oder zusätzlich können Z-Dioden zur Spannungsbegrenzung vorgesehen sein. Des Weiteren ist alternativ oder zusätzlich denkbar, eine Anpassung bzw. eine Fehlanpassung von Impedanzen im Betrieb der Piezoelemente vorzusehen.

In weiterer Ausgestaltung der Erfindung kann der vom US-Empfänger empfangene Ultraschall in ein elektrisches Empfangssignal umwandelbar sein und mit einer Vergleichseinheit (Komparator) mit einem Referenzwert (Referenzspannung, Referenzsignal, Alarmschwelle) verglichen werden. In Abhängigkeit eines Ergebnisses dieses Vergleichs wird von der Vergleichseinheit ein Ausgabesignal ausgegeben, das beispielsweise im Einsatz des Detektors in einem Dialysegerät "Luft" bzw. "keine Luft" anzeigt.

Die Spannungseinrichtung kann an eine Spannungsquelle und an eine Masse zum Ausbilden eines Stromkreises anschließbar sein. Der Stromkreis kann von einem Schalter auf- und zusteuerbar sein, wobei er in Abhängigkeit des Ansteuersignals gesteuert ist.

Vorzugsweise ist zusätzlich zum ersten Referenzwert (Alarmschwelle) ein zweiter Referenzwert (Testschwelle) vorgesehen. Der zweite Referenzwert kann dann, insbesondere zyklisch und für eine vorbestimmte, vergleichsweise kurze, Zeitspanne anstelle des ersten Referenzwerts eingesetzt sein. Somit sind der erste und der zweite Referenzwert am Eingang der Vergleichseinheit vorgesehen. Mit dem kurzzeitigen Vergleich des zweiten Referenzwerts kann zum einen eine Aussage über die Funktionsfähigkeit der Schaltung des Detektors gemacht werden und zum anderen kann eine Aussage über die Kopplung des Mediums, das beispielsweise in einem Schlauch als Flüssigkeit geführt ist, mit dem Detektor getroffen werden. Würde der Detektor fehlerfrei funktionieren, so würde bei dem zweiten Referenzwert ausgangsseitig des Komparators vorzugsweise beispielsweise "Luft" signalisiert sein. Ist dies nicht der Fall, so kann dann entweder ein Schaltungsdefekt vorliegen, oder aber die Kopplung zwischen dem Medium (beispielsweise die im Schlauch geführte Flüssigkeit) und dem US-Sender und/oder US-Empfänger kann sich in unzulässigen Bereichen bewegen. Beispielsweise ist eine unzulässig gute Kopplung oder sogar ein akustischer Kurzschluss möglich, was jeweils zu einer Verfälschung des Ultraschalls zwischen dem US-Senders und US-Empfängers führt. Dies wird beispielsweise durch eingetretene Flüssigkeit zwischen dem US-Sensor, dem Schlauch und dem US-Empfänger verursacht. Es sind jedoch auch Fälle aus der Praxis bekannt, in denen ein Anwender des Detektors ein sog. Ultraschallgel in die Strecke US-Sensor, Schlauch und US-Empfänger einbringt, um vermeintliche Fehlalarme zu beseitigen. Auch in diesen Fällen wird eine Empfindlichkeit des US-Sensors und US-Empfängers herabgesetzt, was dann mit der beschriebenen Methode detektiert werden kann. Das Sicherheitssystem mit den Referenzwerten in Kombination mit der Überwachung des Energieeintrags führt somit zu einem äußerst sicher einsetzbaren Detektor.

Mithilfe der Auswertung des Empfangssignals (Zeit, Amplitude) können Aussagen über die Beschaffenheit eines Medium (Geometrie) in dem zu überwachenden Medium getroffen werden. Mithilfe des Vergleichs des Empfangssignals (Zeit, Amplitude) mit den verschiedenen Referenzwerten können Aussagen über die korrekte Funktion des Sensors sowie die ordnungsgemäße Kopplung zwischen Sensor und zu überwachendem Medium (Schlauch und Flüssigkeit) realisiert werden, so wie es vorstehend bereits erläutert ist. Voraussetzung für die sichere Ableitung des Empfangssignals ist die sichere Kenntnis des Ultraschalls (Sendesignal). Dies kann mit der Realisierung der eingangs beschriebenen Überwachung des Ultraschalls erreicht werden.

Erfindungsgemäß ist ein Dialysegerät oder ein Infusionsgerät vorgesehen, das einen Detektor gemäß einem der vorhergehenden Aspekte aufweist. Zwischen dem US-Sender und dem US-Empfänger kann ein von dem Medium (Blut oder Infusionsflüssigkeit) durchströmbarer Strömungspfad (Schlauch) angeordnet sein. Der Einsatz des Detektors führt zu einer hohen Sicherheit für einen an das Dialysegerät angeschlossenen Patienten, da eine Schädigung des Blutes aufgrund eines hohen Energieeintrags in das Blut vermieden wird.

Die erste Steuereinheit des Dialysegeräts kann zum Regeln und Steuern von Komponenten des Dialysegeräts und des Detektor vorgesehen sein. Die zweite Steuereinheit (Supervisor) kann dann Schutzmaßnahmen für den Patienten einleiten und zusätzlich den Energieeintrag in das Medium überwachen und/oder begrenzen. Hierdurch wird ein Defekt oder eine Fehlfunktion der ersten Steuereinheit sicher von der zweiten Steuereinheit erkannt und es können dann entsprechende Schutzmaßnahmen eingeleitet werden.

Bei fehlerhaftem Energieeintrag kann beim Dialysegerät beispielsweise eine Pumpe oder es können mehrere Pumpen gestoppt sein und/oder es wird eine Schlauchabsperrklemme oder es werden mehrere Schlauchabsperrklemmen verschlossen.

Ein erfindungsgemäßes Verfahren für einen Gasblasen- und/oder Festkörperdetektor auf Ultraschallbasis nach einem der vorhergehenden Aspekte weist folgende Schritte auf:
- senden eines Ultraschalls mit einem Ultraschall(US)-Sender über ein zu überwachendes Medium zu einem Ultraschall(US)-Empfänger des Gasblasen- und/oder Festkörperdetektors und
- überwachen und/oder begrenzen eines Energieeintrag in das Medium aufgrund des Ultraschalls. Hierdurch wird vermieden, dass beispielsweise bei einer Ultraschallbeaufschlagung von Blut dieses beschädigt wird.

Sonstige vorteilhafte Weiterbildungen der Erfindung sind Gegenstand weiterer Unteransprüche.

Im Folgenden werden bevorzugte Ausführungsformen der Erfindung anhand von Zeichnungen näher erläutert. Es zeigen:
Figur 1 in einer schematischen Darstellung einen erfindungsgemäßen Detektor,
Figur 2 in einer schematischen Darstellung ein Ansteuersignal für einen Ultraschall-Sender des Ultraschalldektors,
Figur 3 in einer schematischen Darstellung ein Dialysegerät mit einem Detektor,
Figur 4 in einer schematischen Darstellung eine Vergleichseinheit des Detektors zum Vergleichen eines Empfangssignals mit einem Soll-Wert,
Figur 5 in mehreren Kurven Eingangsgrößen für die Vergleichseinheit aus Figur 4 und
Figur 6 in einem Diagramm die Auswirkungen eines Energieeintrags aufgrund eines Ultraschalls im Blut eines Patienten.

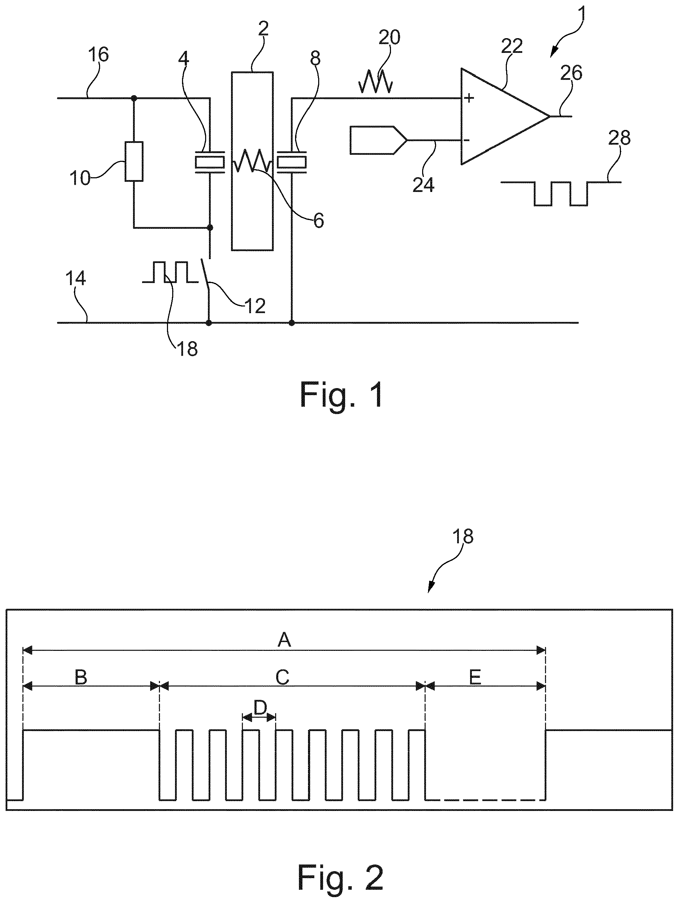

Gemäß Figur 1 ist ein Gasblasen- bzw. Luftblasendetektor 1 (im Folgenden als Detektor bezeichnet) auf Ultraschallbasis zur Detektion von Luft in einem Medium in Form von Blut eines Dialysepatienten vorgesehen, wobei das Blut einen Schlauch 2 durchströmt. Der Detektor 1 weist hierfür einen Ultraschall(US)-Sender 4 in Form eines Piezoelements zum Senden eines Ultraschalls 6 durch den Schlauch 2 auf. Der Ultraschall 6 ist von einem Ultraschall(US)-Empfänger 8 empfangbar. Der US-Sender 4 und der US-Empfänger 8 bilden somit eine Ultraschallstrecke. Zum Aussenden eines Ultraschalls wird der US-Sender 4 mit einer Spannung über eine elektrisch parallel zum US-Sender 4 angeordnete Induktivität 10 beaufschlagt. Der US-Sender 4 und die Induktivität 10 sind über einen Schalter 12 mit einer Masse 14 verbindbar. Des Weiteren sind sie an eine Spannungsquelle 16 angeschlossen. Eine Selbstinduktionsspannung der Induktivität 10, die beim Öffnen des Schalters 12 entsteht, dient dazu, die für den US-Sender 4 benötigte Spannung zur Verfügung zu stellen. Eine Ansteuerung des US-Senders 4 erfolgt dann über ein Ansteuersignal 18. Das Ansteuersignal 18 wird hierbei von einer in der Figur 1 nicht dargestellten Steuereinheit zur Verfügung gestellt. Der Ultraschall 6 wird vom US-Empfänger 8 empfangen und in ein elektrisches Empfangssignal 20 gewandelt. Dieses wird dann von einer Vergleichseinheit 22 (Komparator) mit einer Referenzspannung 24 verglichen und bewertet. An einem Ausgang 26 der Vergleichseinheit 22 steht ein Ausgabesignal 28 zur Verfügung, welches Auskunft darüber gibt, ob sich in der Ultraschallstrecke "Luft" oder "keine Luft" (also nur Flüssigkeit) befindet.

In Figur 2 ist das Ansteuersignal 18 dargestellt, das bei dem erfindungsgemäßen Detektor 1 aus Figur 1 überwacht ist, um wiederum einen Energieeintrag in das in dem Schlauch 2 geführte Blut aufgrund des Ultraschalls 6 zu überwachen und/oder zu begrenzen. Gemäß Figur 2 hat das Ansteuersignal 18 ein Burst-Intervall A, das beispielsweise 50 und 900 µs beträgt. Eine Frequenz des Burst-Intervalls A beträgt beispielsweise 1 bis 15 kHz. Eine Ladezeit B der Induktivität 10 aus Figur 1 beträgt beispielsweise 5 bis 20 µs. Das Burst-Interall A ist dann der Abstand zweier Startzeitpunkte von aufeinanderfolgenden Ladezeiten B.

Nach Ablauf einer Ladezeit B erfolgen bei dem Ansteuersignal 18 eine Anzahl C vonStimuli mit einer US-Anregungsfrequenz D des US-Senders 4 von beispielsweise 1 bis 5MHz. Hierbei sind beispielsweise acht Stimuli (C=8) vorgesehen. Ein Abstand zwischen zwei Stimuli beträgt beispielsweise im Fall einer US-Anregungsfrequenz von 2MHz 500ns. Nach der Anzahl C von Ultraschall Stimuli ist eine Pause E vorgesehen, bis die Ladezeit B erneut startet. Diese Pause beträgt beispielsweise 300 bis 500µs (beispielsweise 481 µs).

Zum Überwachen des Energieeintrags wird nun das Burst-Intervall A und/oder die Ladezeit B und/oder die US-Anregungsfrequenz D und/oder die Anzahl C der Stimuli überwacht. Wird in einem Fehlerfall das Burst-Intervall A verringert, so muss davon ausgegangen werden, dass ein höherer Energieeintrag in den Schlauch 2 mit dem Blut erfolgt. Ebenso muss mit einem höheren Energieeintrag gerechnet werden, wenn sich die Ladezeit B der Induktivität 10 vergrößert. Außerdem muss von einem höheren Energieeintrag in das Blut ausgegangen werden, falls sich in einem denkbaren Fehlerfall die zuvor festgelegte Anzahl C der Stimuli erhöht. Ein höherer Energieeintrag in das Blut kann auch durch eine Veränderung der US-Anregungsfrequenz D hervorgerufen werden. Die angeführten Veränderungen der jeweiligen Parameter können beispielsweise durch ein temporäres oder dauerhaftes Fehlerverhalten der für die Steuereinheit eingesetzten Mikroprozessoren software- oder hardwareseitig entstehen, welche das Ansteuersignal generieren.

Das Ansteuersignal 18 aus Figur 2 steht am Schalter 12 zur Verfügung und kann von dort an eine überwachende Steuereinheit übertragen werden. Die Kenntnis des Energieeintrags führt des Weiteren dazu, dass mithilfe des Empfangssignals 20 (Amplitude und zeitlicher Signalverlauf) auf eine Beschaffenheit eines Gegenstands oder fremden Mediums in dem zu überwachenden Medium, in diesem Fall Blut, geschlossen werden kann. Handelt es sich um ein bekanntes Medium wie in diesem Fall die zu überwachende Luft, so kann dann mithilfe des Empfangssignals 20 auf die Geometrie (Abmessungen, Volumen) geschlossen werden.

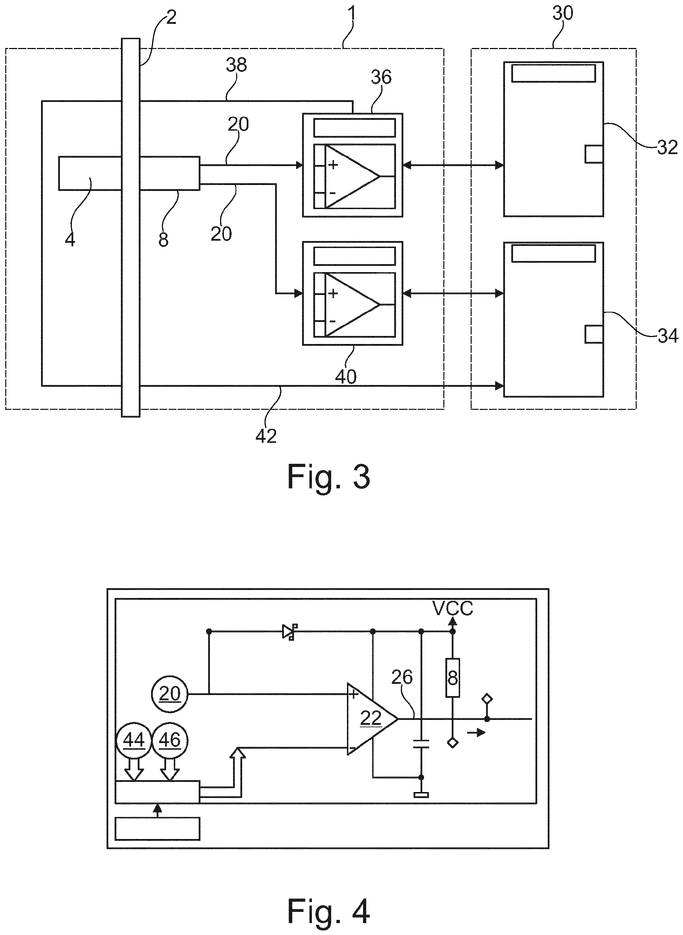

Gemäß Figur 3 ist schematisch zusätzlich zum Detektor 1 ein Dialysegerät 30 dargestellt, das den Detektor 1 aufweist. Das Dialysegerät 30 hat eine erste Steuereinheit 32 und eine zweite Steuereinheit 34 (Supervisor). Die erste Steuereinheit 32 dient zum Regeln und Steuern von Komponenten des Dialysegeräts 30 und somit auch des Detektors 1. Die zweite Steuereinheit 34 dient insbesondere zum Überwachen des Dialysegeräts 30, um einen das Dialysegerät 30 nutzenden Patienten zu schützen.

Die erste Steuereinheit 32 steuert zusammen mit einem Mikroprozessor 36 den US-Sender 4, der den Ultraschall über den Schlauch 2 zum US-Empfänger 8 sendet. Das Empfangssignal 20 wird gemäß Figur 3 zum Mikroprozessor 36 weitergegeben und zu einem Mikroprozessor 40, der mit der zweiten Steuereinheit 34 verbunden ist. Zum Überwachen des Ansteuersignals 18, das über den Signalpfad 38 zum Steuern des US-Senders 4 dient, wird das Ansteuersignal 18 vom US-Sender 4 über einen Signalpfad 42 abgegriffen und einem Kanal der zweiten Steuereinheit 34 des Dialysegeräts 30 zugeführt. Über diesen Kanal werden dann die zu überwachenden Parameter A bis D, siehe Figur 2, kontrolliert. Wird eine Abweichung zu Soll-Parametern von der zweiten Steuereinheit 34 im überwachenden Kanal festgestellt, kann diese Maßnahmen einleiten, welche eine Schädigung des Bluts im Schlauch 2 vermeiden, beenden oder begrenzen. Bei den Maßnahmen handelt es sich beispielsweise um ein Stoppen einer oder mehrerer Pumpen und/oder um das Verschließen einer oder mehrerer Schlauchabsperrklemmen .

Gemäß Figur 4 kann mit der Überwachung der Sequenz des Ansteuersignals 28 aus Figur 1 ein Verfahren zur Auswertung des Empfangssignals 20 kombiniert werden. Dieses dient zur Detektion von Fehlern des US-Senders 4 oder des US-Empfängers 8 und zur Detektion von Veränderungen einer Kopplung in der Sensorstrecke. Gemäß Figur 4 wird das Empfangssignal 20 von der Vergleichseinheit 22 oder einer weiteren Vergleichseinheit mit einem Referenzwert 44 verglichen, das als Alarmschwelle (AS) bezeichnet werden kann. Die Vergleichseinheit 22 zeigt dann abhängig vom Empfangssignal 20, wie vorstehend bereits erläutert, "Luft" oder "keine Luft" an seinem Ausgang 26 an. Wird nun zyklisch für eine kurze Zeit vom ersten Referenzwert 44 auf einen zweiten Referenzwert 46, der als Testschwelle (TS) am Eingang der Vergleichseinheit 22 dient, gewechselt, so wird das Empfangssignal 20 mit dem zweiten Referenzwert 46 verglichen. Hierdurch kann dann zum einen eine Aussage über die Funktionsfähigkeit der in Figur 1 gezeigten Schaltung und zum anderen Aussagen über die Kopplung zwischen dem Schlauch 2 und dem US-Sender 4 bzw. dem US-Empfänger 8 getroffen werden. Wird bei dem Heranziehen des zweiten Referenzwerts 46 am Ausgang 26 nicht wie erwartet "Luft" signalisiert, kann entweder ein Schaltungsdefekt vorliegen, oder aber die Kopplung zwischen dem Schlauch 2 und dem US-Sender 4 und/oder dem US-Empfänger 8 kann sich in unzulässigen Bereichen bewegen.

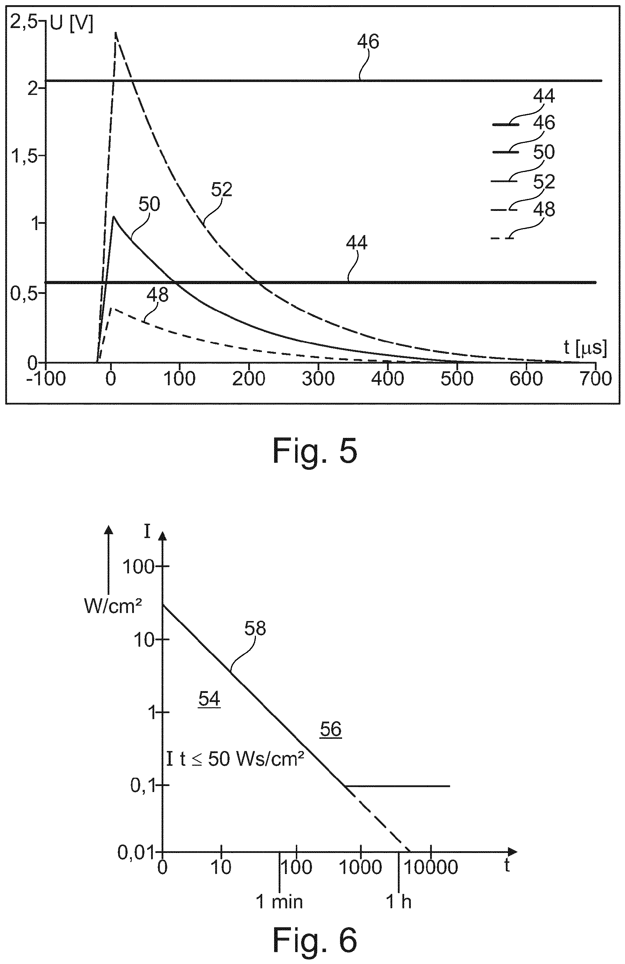

Gemäß Figur 5 ist anhand von mehreren Kurven ein Vergleich mit dem ersten Referenzwert 44 und mit dem zweiten Referenzwert 46 gezeigt. Das in Figur 5 gezeigte Diagramm hat eine Ordinate, die eine Spannung in V anzeigt, und eine Abszisse, die eine Zeit in µs anzeigt. Die Spannung reicht hierbei von 0 bis 2,5 V und die Zeit von - 100 bis 700 µs. Der erste Referenzwert 44 hat eine Spannung, die zwischen 0,5 und 1 V liegt und der zweite Referenzwert 46 hat eine Spannung, die zwischen 2 und 2,5 V liegt, wobei ein jeweiliger Referenzwert 44, 46 etwa konstant ist. Eine Kurve 48, die gemäß Figur 5 unterhalb des ersten Referenzwerts 44 liegt, stellt das Empfangssignal 20 aus Figur 1 dar, das sich im fehlerfreien Betrieb ergibt, wenn sich "Luft" im Schlauch 2 befindet. In diesem Fall würde ein Vergleich zwischen dem ersten Referenzwert 44 und auch dem zweiten Referenzwert 46 dazu führen, dass am Ausgang 26 der Vergleichseinheit 22 "Luft" angezeigt wird. Eine Kurve 50 in Figur 5, die den ersten Referenzwert 44 schneidet, zeigt das Empfangssignal 20, wenn sich im fehlerfreien Betrieb "keine Luft" im Schlauch 2 befindet. In diesem Fall wird "keine Luft" am Ausgang 26 ausgegeben. Bei einem Vergleich mit dem zweiten Referenzwert 46 würde "Luft" angezeigt werden. Eine weitere Kurve 52, die den zweiten Referenzwerts 46 schneidet, zeigt das Empfangssignal 20 bei einer fehlerhaften Kopplung an. Wird die Kurve 52 mit dem ersten Referenzwert 44 verglichen, so würde festgestellt werden, dass "keine Luft" vorliegt, da die Kurve 52 auch den ersten Referenzwerts 44 schneidet. Wird allerdings der zweite Referenzwert 46 herangezogen, so müsste im fehlerfreien Betrieb "Luft" angezeigt werden, da die Kurven 48, 50 für das fehlerfreie Empfangssignal 20 unterhalb des zweiten Referenzwerts 46 liegen. Da in einem Fehlerfall die Kurve 52 allerdings die Gerade des zweiten Referenzwerts 46 schneidet, wird "keine Luft" gemeldet, woraus auf einen fehlerbehafteten Betrieb geschlossen werden kann.

Gemäß Figur 6 ist ein Diagramm dargestellt, das auf der Ordinate eine Intensität I in W/cm² der Energie des Ultraschalls 6 aus Figur 1 und auf der Abszisse eine Einwirkungsdauer t in s des Ultraschalls 6 aufzeigt. Die Intensität I ist hierbei logarithmisch zwischen 0,01 bis 100 und die Einwirkungsdauer logarithmisch zwischen 0 und 10 000 aufgezeigt. Hierbei sind ein sicherer Bereich 54 und ein möglicher Schädigungsbereich 56 dargestellt, die von einer Kurve 58 getrennt sind. Wenn das Produkt aus Intensität und Einwirkungsdauer (I*t) im Sicherheitsbereich 54 liegt, das heißt, das Produkt ≤ 50 Ws/cm² ist, so liegt keine Schädigung des Bluts im Schlauch 2 aus Figur 1 vor. Ist das Produkt dagegen größer und liegt im Schädigungsbereich 56, so kann eine Schädigung des Bluts, beispielsweise durch Auftreten einer Hämolyse, stattfinden.

Offenbart ist ein Detektor mit einem Ultraschall-Sender zum Senden eines Ultraschalls durch ein zu prüfendes Medium hindurch, wobei der Ultraschall von einem Ultraschall-Empfänger empfangbar ist. Ein Energieeintrag des Ultraschalls in das zu überwachende Medium kann hierbei begrenzt und/oder überwacht und/oder angepasst werden.

### Bezugszeichenliste

- 1: Detektor
- 2: Schlauch
- 4: US-Sender
- 6: Ultraschall
- 8: US-Empfänger
- 10: Induktivität
- 12: Schalter
- 14: Masse
- 16: Spannungsquelle
- 18: Ansteuersignal
- 20: Empfangssignal
- 22: Vergleichseinheit
- 24: Referenzspannung
- 26: Ausgang
- 28: Ausgabesignal
- 30: Dialysegerät
- 32: erste Steuereinheit
- 34: zweite Steuereinheit
- 36: Mikroprozessor
- 38: Signalpfad
- 40: Mikroprozessor
- 42: Signalpfad
- 44: erster Referenzwert
- 46: zweiter Referenzwert
- 48: Kurve (Luft im fehlerfreien Betrieb)
- 50: Kurve (keine Luft im fehlerfreien Betrieb)
- 52: Kurve (fehlerbehafteter Betrieb)
- 54: sicherer Bereich
- 56: Schädigungsbereich
- A: Burst-Intervall
- B: Ladezeit
- D: Ultraschall Anregungsfrequenz
- C: Anzahl Stimuli
- E: Pause

## Patentansprüche

1. Gasblasen und/oder Festkörperdetektor auf Ultraschallbasis mit einem Ultraschall(US)-Sender zum Senden eines Ultraschalls (6) durch ein zu überwachendes Medium hindurch zu einem Ultraschall(US)-Empfänger (8) des Gasblasen- und/oder Festkörperdetektors, einer Vergleichseinheit (22) zum Vergleichen eines Empfangssignals (20) mit einem Referenzwert (24) und einer oder mehreren Pumpen und/oder einer oder mehreren Schlauchabsperrklemmen, wobei ein Überwachungs- und/oder Begrenzungsmittel bzw. -einrichtung vorgesehen ist, das/die dazu angepasst ist, einen Energieeintrag in das Medium aufgrund des Ultraschalls zu überwachen und/oder zu begrenzen, wobei
das Überwachungs- und/oder Begrenzungsmittel dazu angepasst ist, als eine zweite Steuereinheit (34, 40) die Überwachung des Energieeintrags durch eine Überwachung einer Ultraschallgenerierung zu erwirken, **dadurch gekennzeichnet, dass**
der US-Sender (4) über eine erste Steuereinheit (32, 36) durch ein Ansteuersignal (18) gesteuert ist, wobei zum Überwachen des Energieeintrags das Ansteuersignal (18) abgegriffen ist oder wird, wobei
eine Spannungseinrichtung (10) vorgesehen ist und wobei der US-Sender (4) zum Aussenden des Ultraschalls (6) mit einer Spannung der Spannungseinrichtung (10) beaufschlagt wird, wobei
ein Burst-Intervall (A) des Ansteuersignals (18) überwacht ist, das eine Zeitspanne zwischen Startzeitpunkten zweier aufeinander folgender Ladevorgänge der Spannungseinrichtung (10) darstellt, und/oder wobei eine Ladezeit (B) der Spannungseinrichtung (10) überwacht ist, und/oder wobei eine Anzahl (C) von, vom US-Sender (4) ausgegebenen Stimuli pro Burst-Intervall (A) überwacht sind.

2. Gasblasen- und/oder Festkörperdetektor auf Ultraschallbasis nach Anspruch 1, wobei das Ansteuersignal (18) von der zweiten Steuereinheit (34, 40) überwacht ist oder wird.

3. Gasblasen- und/oder Festkörperdetektor auf Ultraschallbasis nach einem der vorhergehenden Ansprüche, wobei der US-Sender (4) und der US-Empfänger (8) durch jeweils ein Piezoelement ausgebildet sind.

4. Gasblasen- und/oder Festkörperdetektor auf Ultraschallbasis nach Anspruch 1, wobei die zweite Steuereinheit (34, 40) dazu eingerichtet ist, dass bei Erhöhung der Ladezeit (B) ein Fehlersignal ausgebbar ist, und/oder dass bei einer Verringerung des Burst-Intervalls (A) ein Fehlersignal ausgebbar ist, und/oder dass bei einer Erhöhung der Anzahl (C) der Stimuli ein Fehlersignal ausgebbar ist.

5. Gasblasen- und/oder Festkörperdetektor auf Ultraschallbasis nach einem der vorhergehenden Ansprüche, wobei die zweite Steuereinheit (34, 40) dazu eingerichtet ist, dass bei einem fehlerhaften Energieeintrag Maßnahmen eingeleitet werden, die den Energieeintrag begrenzen oder beenden.

6. Gasblasen- und/oder Festkörperdetektor auf Ultraschallbasis nach Anspruch 5, wobei der fehlerhafte Energieeintrag durch Hardwaremaßnahmen mittels einem oder mehrerer Bauteile begrenzt ist.

7. Gasblasen- und/oder Festkörperdetektor auf Ultraschallbasis nach einem der vorhergehenden Ansprüche, wobei der vom US-Empfänger (8) empfangene Ultraschall (6) in das elektrisches Empfangssignal (20) umwandelbar ist und über die Vergleichseinheit (22) mit dem Referenzwert (24) vergleichbar ist, wobei in Abhängigkeit des Vergleichs in der Vergleichseinheit (22) ein Ausgabesignal (28) ausgegeben ist, das ein Detektionsergebnis anzeigt.

8. Gasblasen- und/oder Festkörperdetektor auf Ultraschallbasis nach Anspruch 7, wobei zusätzlich zum ersten Referenzwert (44) ein zweiter Referenzwert (46) vorgesehen ist, wobei der zweite Referenzwert (46) zyklisch und für eine vorbestimmte Zeitspanne eingesetzt ist.

9. Blutbehandlungsgerät, insbesondere Dialysegerät, mit einem Gasblasen- und/oder Festkörperdetektor auf Ultraschallbasis gemäß einem der vorhergehenden Ansprüche 1 bis 8, wobei ein vom Medium durchströmbarer Strömungspfad zwischen dem US-Sender (4) und dem US-Empfänger (8) angeordnet ist.

10. Blutbehandlungsgerät nach Anspruch 9, wobei die erste Steuereinheit (32) zum Regeln und Steuern des Blutbehandlungsgeräts(30) vorgesehen ist, und wobei das Überwachungs- und/oder Begrenzungsmittel, insbesondere die zweite Steuereinheit (34) zum Einleiten von Schutzmaßnahmen für einen Patienten vorgesehen ist, wobei das Überwachungs- und/oder Begrenzungsmittel, insbesondere die zweite Steuereinheit (34) den Energieeintrag in das Medium überwacht und/oder begrenzt.

11. Blutbehandlungsgerät nach Anspruch 9 oder 10, wobei bei einem fehlerhaften Energieeintrag beim Blutbehandlungsgerät (30) die eineoder mehrere Pumpen gestoppt sind und/oder die eine oder mehrere Schlauchabsperrklemmen verschlossen sind.

12. Verfahren für einen Gasblasen und/oder Festkörperdetektor auf Ultraschallbasis nach einem der Ansprüche 1 bis 8 mit den Schritten:
- senden eines Ultraschalls mit einem Ultraschall(US)-Sender durch ein zu überwachendes Medium hindurch zu einem Ultraschall(US)-Empfänger des Gasblasen- und/oder Festkörperdetektors und
- überwachen und/ oder begrenzen eines Energieeintrags in das Medium aufgrund des Ultraschalls.

## Claims

1. An ultrasound-based air bubble and/or solid detector comprising an ultrasonic (US) transmitter for transmitting an ultrasound through a medium to be monitored to an ultrasonic (US) receiver (8) of the air bubble and/or solid detector, a comparing unit (22) for comparing a received signal (20) to a reference value (24) and one or more pumps and/or one or more hose shut-off clamps, wherein a monitoring and/or limiting means is provided which is adapted to monitor and/or limit energy input into the medium on the basis of the ultrasound, wherein
the monitoring and/or limiting means, as a second control unit (34, 40), is adapted to cause the monitoring of the energy input by monitoring an ultrasound generation, **characterized in that**
the US transmitter (4) is controlled via a first control unit (32, 36) by a control signal (18), the control signal (18) being tapped for monitoring the energy input, wherein
a voltage means (10) is provided and voltage of the voltage means (10) is applied to the US transmitter (4) for emitting the ultrasound (6), wherein
a burst interval (A) of the control signal (18) representing a time interval between starting times of two successive charging operations of the voltage means (10) is monitored and/or wherein a charging time (B) of the voltage means (10) is monitored and/or wherein a number (C) of stimuli output by the US transmitter (4) per burst interval (A) is monitored.

2. The ultrasound-based air bubble and/or solid detector according to claim 1, wherein the control signal (18) is monitored by the second control unit (34, 40).

3. The ultrasound-based air bubble and/or solid detector according to any one of the preceding claims, wherein the US transmitter (4) and the US receiver (8) are each constituted by a piezo element.

4. The ultrasound-based air bubble and/or solid detector according to claim 1, wherein the second control unit (34, 40) is configured such that an error signal can be output upon increase of the charging time (B), and/or that an error signal can be output upon reduction of the burst interval (A), and/or that an error signal can be output upon increase in the number (C) of stimuli.

5. The ultrasound-based air bubble and/or solid detector according to any one of the preceding claims, wherein the second control unit (34, 40) is configured such that measures that limit or terminate the energy input are initiated in the case of faulty energy input.

6. The ultrasound-based air bubble and/or solid detector according to claim 5, wherein the faulty energy input is limited by hardware measures by one or more components.

7. The ultrasound-based air bubble and/or solid detector according to any one of the preceding claims, wherein the ultrasound (6) received by the US receiver (8) can be converted into the electric received signal (20) and is comparable to the reference value (24) via the comparing unit (22), an output signal (28) indicating a detection result being output in the comparing unit (22) in response to the comparison.

8. The ultrasound-based air bubble and/or solid detector according to claim 7, wherein a second reference value (46) is provided in addition to the first reference value (44), wherein the second reference value (46) is used cyclically and for a predetermined time interval.

9. A blood treatment apparatus, in particular a dialysis apparatus, comprising an ultrasound-based air bubble and/or solid detector according to any one of the preceding claims 1 to 8, wherein a flow path through which the medium can flow is arranged between the US transmitter (4) and the US receiver (8).

10. The blood treatment apparatus according to claim 9, wherein the first control unit (32) is provided for regulating and controlling the blood treatment apparatus (30) and wherein the monitoring and/or limiting means, in particular the second control unit (34) is provided for initiating protective measures for a patient, the monitoring and/or limiting means, in particular the second control unit (34), monitoring and/or limiting the energy input into the medium.

11. A blood treatment apparatus according to claim 9 or 10, wherein in the case of faulty energy input in the blood treatment apparatus (30) said one or more pumps are stopped and/or said one or more hose shut-off clamps are closed.

12. A method for an ultrasound-based air bubble and/or solid detector, according to any one of the claims 1 to 8, comprising the steps of:
- transmitting an ultrasound by an ultrasonic (US) transmitter through a medium to be monitored to an ultrasonic (US) receiver of the air bubble and/or solid detector and
- monitoring and/or limiting energy input into the medium on the basis of the ultrasound.

## Revendications

1. Détecteur de bulles de gaz et/ou de corps solides basé sur des ultrasons avec un émetteur d'ultrason (US) destiné à émettre un ultrason (6) à travers un milieu à surveiller en direction d'un récepteur (8) d'ultrason (US) du détecteur de bulles de gaz et/ou de corps solides, une unité de comparaison (22) destinée à comparer un signal de réception (20) avec une valeur de référence (24) et une ou plusieurs pompes et/ou un ou plusieurs éléments de serrage d'arrêt de tuyau, dans lequel un moyen ou dispositif de surveillance et/ou de limitation est prévu, qui est adapté à surveiller et/ou limiter un apport d'énergie dans le milieu du fait de l'ultrason, dans lequel
le moyen de surveillance et/ou de limitation est adapté à obtenir en tant que seconde unité de commande (34, 40) la surveillance de l'apport d'énergie par une surveillance d'une génération d'ultrason, **caractérisé en ce que**
l'émetteur d'ultrason (4) est commandé par un signal de commande (18) par l'intermédiaire d'une première unité de commande (32, 36), dans lequel pour surveiller l'apport d'énergie, le signal de commande (18) est ou devient détecté, dans lequel
un dispositif de tension (10) est prévu et dans lequel l'émetteur d'ultrason (4), pour l'émission de l'ultrason (6), est soumis à l'effet d'une tension du dispositif de tension (10), dans lequel
un intervalle de salve (A) du signal de commande (18) est surveillé, qui représente un laps de temps entre des instants de démarrage de deux processus de charge successifs du dispositif de tension (10), et/ou dans lequel un temps de charge (B) du dispositif de tension (10) est surveillé, et/ou dans lequel une pluralité (C) de stimuli émis par l'émetteur d'ultrason (4) par intervalle de salve (A) sont surveillés.

2. Détecteur de bulles de gaz et/ou de corps solides basé sur des ultrasons selon la revendication 1, dans lequel le signal de commande (18) est ou devient surveillé par la deuxième unité de commande (34, 40).

3. Détecteur de bulles de gaz et/ou de corps solides basé sur des ultrasons selon l'une quelconque des revendications précédentes, dans lequel l'émetteur d'ultrason (4) et le récepteur d'ultrason (8) sont réalisés par respectivement un élément piézoélectrique.

4. Détecteur de bulles de gaz et/ou de corps solides basé sur des ultrasons selon la revendication 1, dans lequel la deuxième unité de commande (34, 40) est conçue pour que, lors de l'augmentation du temps de charge (B), un signal d'erreur puisse être émis, et/ou en ce que lors d'une réduction de l'intervalle de salve (A), un signal d'erreur puisse être émis, et/ou en ce que, lors d'une augmentation du nombre (C) des stimuli, un signal d'erreur puisse être émis.

5. Détecteur de bulles de gaz et/ou de corps solides basé sur des ultrasons selon l'une quelconque des revendications précédentes, dans lequel la seconde unité de commande (34, 40) est conçue pour que, lors d'un d'apport d'énergie défectueux, des mesures soient prises, qui limitent ou terminent l'apport d'énergie.

6. Détecteur de bulles de gaz et/ou de corps solides basé sur des ultrasons selon la revendication 5, dans lequel l'apport d'énergie défectueux est limité par des mesures matérielles au moyen d'un ou de plusieurs composants.

7. Détecteur de bulles de gaz et/ou de corps solides basé sur des ultrasons selon l'une quelconque des revendications précédentes, dans lequel l'ultrason (6) reçu par le récepteur d'ultrason (8) peut être converti en le signal de réception électrique (20) et peut être comparé à la valeur de référence (24) par l'intermédiaire de l'unité de comparaison (22), dans lequel un signal de sortie (28), qui indique un résultat de détection, est émis en fonction de la comparaison dans l'unité de comparaison (22).

8. Détecteur de bulles de gaz et/ou de corps solides basé sur des ultrasons selon la revendication 7, dans lequel, en plus de la première valeur de référence (44), une deuxième valeur de référence (46) est prévue, dans lequel la deuxième valeur de référence (46) est utilisée de manière cyclique et pendant un laps de temps prédéfini.

9. Appareil de traitement du sang, en particulier appareil de dialyse, avec un détecteur de bulles de gaz et/ou de corps solides basé sur des ultrasons selon l'une quelconque des revendications précédentes 1 à 8, dans lequel un trajet d'écoulement pouvant être parcouru par le milieu est disposé entre l'émetteur d'ultrason (4) et le récepteur d'ultrason (8).

10. Appareil de traitement du sang selon la revendication 9, dans lequel la première unité de commande (32) est destinée à réguler et commander l'appareil de traitement du sang (30), et dans lequel le moyen de surveillance et/ou limitation, en particulier la deuxième unité de commande (34), est destiné à prendre des mesures de protection pour un patient, dans lequel le moyen de surveillance et/ou limitation, en particulier la deuxième unité de commande (34), surveille et/ou limite l'apport d'énergie dans le milieu.

11. Appareil de traitement du sang selon la revendication 9 ou 10, dans lequel, lors d'un apport d'énergie défectueux pour l'appareil de traitement du sang (30), l'une des ou les différentes pompes sont arrêtées et/ou l'un des ou les différents éléments de serrage d'arrêt de tuyau flexible sont fermés.

12. Procédé pour un détecteur de bulles de gaz et/ou de corps solides basé sur des ultrasons selon l'une quelconque des revendications 1 à 8 comprenant les étapes :
- d'envoi d'un ultrason avec un émetteur d'ultrason (US) à travers un milieu à surveiller en direction d'un récepteur d'ultrason (US) du détecteur de bulles de gaz et/ou de corps solides basé sur des ultrasons et
- de surveillance et/ou limitation d'un apport d'énergie dans le milieu du fait de l'ultrason.
